# EUROPEAN PATENT APPLICATION

(11) **EP 1 661 518 A1**
(43) Date of publication of application: **31.05.2006**
(21) Application number: 05105372.6
(22) Date of filing: 17.06.2005
(51) Int. Cl.: A61B 6/14, G03B 42/04, A61C 19/00, A61C 19/02, B65D 61/00, B65D 85/30, B65D 85/38, G01T 1/29

(54) **Removeable elastomeric impact protection cover for wireless dental radiographic sensor unit**

(30) Priority: 26.11.2004 IT BO20040735
(71) Applicant: ELCA TECHNOLOGIES S.r.l., 40026 Imola (IT)
(72) Inventor: Nanni, Eros, 40023 Castel Guelfo Di Bologna (IT); Geminiani, Andrea, 40026 Imola (IT)
(74) Representative: Jorio, Paolo

(57) **Abstract**

In a unit for acquiring dental radiographic images, a radiographic sensor (5) is adapted to acquire a dental radiographic image generated by an X-ray emitter device (2), and is provided with a protective container (19), which is made of elastically deformable material, and presents a guide (24) adapted to being slidingly engaged by the sensor (5) itself.

## Description

The present invention relates to a unit for acquiring dental radiographic images.

In dentistry, it is known a unit for acquiring dental radiographic images of the type comprising an X-ray emitter device; and a radiographic sensor adapted to acquire a dental radiographic image generated by the emitter device and to transmit the acquired image to a computer adapted to store and display the image itself.

The known dental radiographic image acquiring units of the type described above present some drawbacks mainly deriving from the fact that, when inactive, the sensor may fail and be damaged relatively easily due to accidental shocks and/or falls.

It is the object of the present invention to provide a unit for acquiring dental radiographic images which is free from the abovementioned drawbacks.

According to the present invention, there is provided a unit for acquiring dental radiographic images as claimed in the attached claims.

The present invention will now be described with reference to the attached drawings, which show a non-limitative example of embodiment, in which:
figure 1 is a perspective view of a preferred embodiment of the unit of the present invention;
figure 2 is a perspective view of a detail of the unit in figure 1; and
figure 3 is an exploded perspective view of the detail in figure 2.

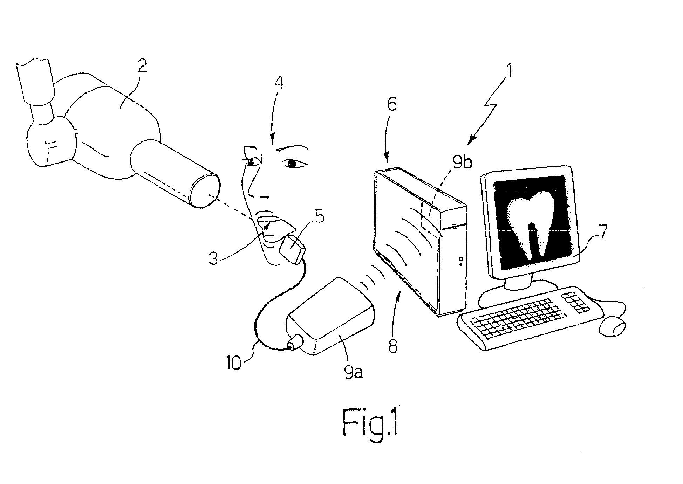

With reference to figure 1, numeral 1 indicates, as a whole, a unit for acquiring and transmitting dental radiographic images comprising an emitter device 2 adapted to address a beam of X-rays towards the oral cavity 3 of a patient 4; a radiographic sensor 5 adapted to acquire a radiographic image of the oral cavity 3 generated by the device 2; a computer 6 adapted to store and display on a respective monitor 7 the radiographic image acquired by the sensor 5; and a transmission device 8 adapted to reciprocally connect the sensor 5 to the computer 6.

The device 8 comprises two electronic interfaces 9, one of which (indicated hereinafter by numeral 9a) is connected to the sensor 5 via a connection cable 10 and is powered by at least one rechargeable battery (not shown) and the other (indicated hereinafter by numeral 9b) is associated to the computer 6.

The interfaces 9a and 9b are adapted to communicate with each other, in the case at hand, via radio signals to transfer the radiographic image acquired by the sensor 5 to the computer 6. According to a variant not shown, the two interfaces 9 are connected together by an electrical connection cable.

Preferably, each interface 9 is an interface using a digital standard of the commercial type working in a frequency range from 300 MHz to 10 GHz. The electronic interface 9 connected to the computer 6 works according to a standard image management commercial protocol (for example according to the protocol called "TWAIN" or according to the protocol called "STI" abbreviation of "Still Image application programming interface").

The sensor 5 comprises a CCD sensor, or alternatively, a CMOS sensor and a scintillator device not shown coupled to the CCD or CMOS sensor itself.

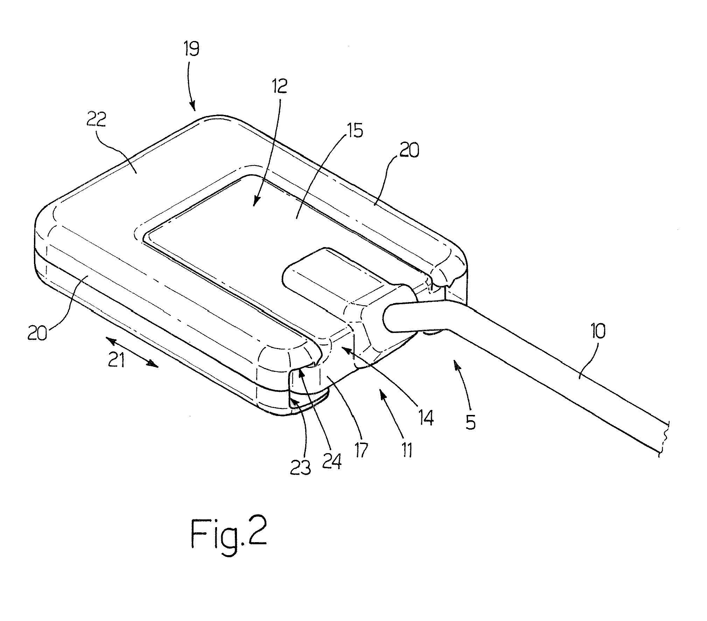

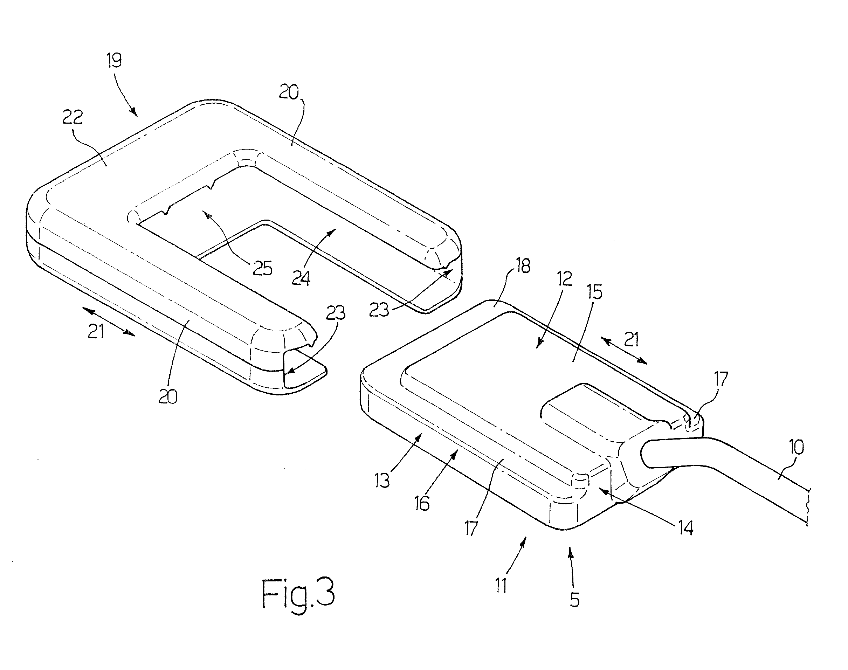

With reference to figures 2 and 3, the sensor 5 comprises a plate 11, which presents an substantially parallelepipedal shape, and is limited by two bigger side faces 12 substantially reciprocally parallel, and by two shorter side faces 14 substantially reciprocally parallel and perpendicular to the faces 12 and 13.

The plate 11 is discharged along a side edge of a face 12 in the region of faces 13 and of a face 14 so to define a central portion 15 to which is fastened the cable 10 and a side edge 16, essentially U shaped, extending around the portion 15, presenting a smaller thickness than the thickness of the portion 15, and comprising two reciprocally parallel longitudinal sections 17 and a transversal section 18 extending between the sections 17 transversally to the sections 17 themselves.

The unit 1 comprises, finally, a protective container 19, which is made of elastically deformable material and is adapted to house inside the plate 11, when the sensor 5 is not active, to protect the sensor 5 from accidental shocks and/or falls. In the case in point, the container 19 is made of an elastomer with hardness comprised in the range from 25 to 100 Shore A, preferably but not necessarily a hardness equal to 70 Shore A.

The container 19 is essentially U shaped, and comprises two arms 20 parallel to each other and to a direction 21, and a stop element 22 extending between the arms 20 transversally with respect to the direction 21 itself.

Each arm 20 presents a groove 23, which is parallel to the direction 21, is obtained along an edge of the respective arm 20 facing the other arm 20, and defines, with the groove 23 of the other arm 20, a guide 24 adapted to be slidingly engaged by the longitudinal sections 17 of the side edge 16 of the plate 11.

The element 22 presents a groove 25, extending transversally to direction 21, which is obtained at an inner edge of the element 22, and is adapted to be slidingly engaged by the transversal section 18 of the side edge 16 of the plate 11 following the insertion of the plate 11 in the container 19.

## Claims

1. A unit for acquiring dental radiographic images, the unit comprising an X-ray emitter device (2); and a radiographic sensor (5) for acquiring a dental radiographic image generated by the emitter device (2); and being **characterised in that** it further comprises a protective container (19) made of elastically deformable material and adapted to housing the sensor (5) therein; the container (19) comprising a guide (24), which extends in a determined direction (21), and is adapted to being slidingly engaged by the sensor (5) itself.

2. A unit according to claim 1, wherein said container (19) is made of elastomeric material.

3. A unit according to claim 2, wherein said elastomeric material presents a hardness comprised in the range from 25 to 100 Shore A.

4. A unit according to claim 2 or 3, wherein said elastomeric material presents a hardness substantially equal to 70 Shore A.

5. A unit according to any of the previous claims, wherein the container (19) is essentially U shaped, and comprises two arms (20), which are substantially parallel to each other and to said direction (21), and define said guide (24).

6. A unit according to claim 5, wherein the arms (20) present respective grooves (23), which extend parallelly to the direction (21), face each other, and define said guide (24).

7. A unit according to claim 6, wherein the sensor (5) comprises a plate (11), whose shape is substantially parallelepipedal, and is provided with two opposite longitudinal edges (17) substantially parallel to said direction (21) and adapted to slidingly engage said grooves (23).

8. A unit according to any of the claims from 5 to 7, wherein the container (19) comprises a stop element (22), which extends between the two arms (20) transversally to said direction (21), and presents a further groove (25) adapted to being slidingly engaged by the sensor (5) following a inserting shift of the sensor (5) in the container (19).

9. A unit according to claim 8, wherein the sensor (5) comprises a plate (11), whose shape is essentially parallelepipedal, and is provided with two transversal opposite edges (18) substantially transversal to said direction (21); a first said transversal edge (18) being adapted to slidingly engage said further groove (25) following a shift of the sensor (5) in the container (19).

10. A unit according to claim 9 and also comprising a device for storing and displaying (6, 7) said image, and two communication devices (9) associated to the sensor (5) and, respectively, to the storage and visualisation device (6, 7) for transmitting the image from the sensor (5) to the storage and visualisation (6, 7) device itself; the sensor (5) and the respective communication device (9) being reciprocally connected by a connection cable (10) connected to the sensor (5) at a second said transversal edge.
